# EUROPÄISCHE PATENTSCHRIFT

(11) **EP 2 552 437 B1**
(45) Veröffentlichungstag und Bekanntmachung des Hinweises auf die Patenterteilung: **24.04.2019**
(21) Anmeldenummer: 10757715.7
(22) Anmeldetag: 02.09.2010
(51) Int. Cl.: A61K 31/195, A61K 31/198, A61Q 17/04, A61K 8/41, A61K 8/49

(54) **MIKROBIOLOGISCH STABILE ANWENDUNGSFREUNDLICHE ZUBEREITUNGEN MIT ANIONISCHEN UV-FILTERN**
MICROBIOLOGICALLY STABLE, EASILY APPLICABLE PREPARATIONS COMPRISING ANIONIC UV-FILTERS
PRÉPARATIONS MICROBIOLOGIQUEMENT STABLES ET D'UTILISATION AISÉE, CONTENANT DES FILTRES UV ANIONIQUES

(30) Priorität: 29.03.2010 DE 102010013276
(43) Veröffentlichungstag der Anmeldung: 06.02.2013
(73) Patentinhaber: Beiersdorf AG, 20253 Hamburg (DE)
(72) Erfinder: KÖHLER, Manuela, 22147 Hamburg (DE); SCHULZ, Jens, 25462 Rellingen (DE); FILBRY, Alexander, 22459 Hamburg (DE); KRÖPKE, Rainer, 22869 Schenefeld (DE); WAGNER, Antonia, 22525 Hamburg (DE); ARGEMBEAUX, Horst, 21465 Wentorf (DE); PILZNER, Anke, 22459 Hamburg (DE)
(86) Internationale Anmeldenummer: PCT/EP2010/005379
(87) Internationale Veröffentlichungsnummer: WO 2011/124245

(56) Entgegenhaltungen:
- EP-A2- 1 153 544
- WO-A1-02/069710
- WO-A1-03/013454
- WO-A2-2006/125098
- US-A1- 2007 082 018
- DATABASE GNPD [Online] MINTEL; 30 June 2009 (2009-06-30), "Clean-Dry Shampoo", Database accession no. 1134103

## Beschreibung

Die Erfindung umfasst kosmetische oder dermatologische Zubereitungen gemäß Anspruch 1.

Emulsionen werden in vielen Bereichen des täglichen Lebens verwendet. Mit diesen Emulsionen werden verschiedene Wirkstoffe an ihren Bestimmungsort gebracht. Dabei kann es sich um unter anderem Farbe, Lacke, Lebensmittel, Reinigungsmittel und Kosmetika handeln. In allen Fällen enthalten die Zubereitungen Substanzen die man gezielt an einen Wirkort bringen möchte, ohne dass diese auf dem Weg dahin mit anderen Substanzen Kontakt bekommen die unter Umständen zum Beispiel als Reaktionspartner dienen können. Ein anderer Grund Substanzen durch eine Zubereitung an einen Wirkort bringen zu wollen kann die Tatsache sein, das die Substanz pur oder in einem Lösemittel alleine nicht Anwenderfreundlich zu applizieren oder zu dosieren ist. Dies kann zu ungewollter Fehldosierung oder zu ungleichmäßiger Dosierung führen. Als ein Beispiel aus der Kosmetik kann hier der Selbstbräuner DHA (Dehydroxyaceton) genannt werden. Wird dieser ungleichmäßig oder überdosiert aufgetragen, erhält der Konsument nicht das gewünschte gleichmäßige Bräunungsergebnis, sondern ein fleckiges und gegebenenfalls auch orangenes Hautbild.

In der kosmetischen Industrie werden viele Wirkstoffe für diverse Wünsche und Nöte der Verbraucher eingesetzt. Einige dieser Wirkstoff sind sehr formulierungsfreundlich und unkompliziert. Leider ist dies die Ausnahme. Die meisten Wirkstoffe tendieren dazu sich mit der Zeit unter verschiedensten Einflüssen zu verändern und in den meisten Fällen sich zu unwirksamen Substanzen abzubauen. Im ungünstigsten Fall führt der Abbau neben dem Wirkverlust auch noch zu ungünstigen Abbauprodukten. Diese Abbauprodukte können farbig sein, unangenehm riechen oder ein Reizpotential für die Haut beinalten. Einige dieser Wirkstoffe tendieren nicht zum Abbau aber zur Komplexbildung mit anderen Stoffen in der Formulierung. Dies führt im Allgemeinen zu Inaktivierung und damit zum Verlust der Leistungstärke des Produktes. Dies geschieht insbesondere wenn eine anionisch geladene und einen kationisch geladene Substanz in einer Formulierung, insbesondere in einer Phase der Formulierung vorliegen. Aufgrund der Coulomb'schen Anziehung finden sich diese Moleküle sehr schnell und bilden Komplexe. Diese Komplexe sind im ungünstigen Fall zusätzlich zur Inaktivität auch schwerlöslich und dienen in der Formulierung als Kristallisationskeim. Dies kann zu auf der Haut spürbaren Kristallen führen, welche vom Verbraucher gespürt werden können. Die Inaktivierung und die eventuelle Kristallisation gilt es bei qualitativ hochwertigen Produkte zu vermeiden.

Kosmetische Zubereitungen müssen darüber hinaus langzeitstabil gegen mikrobielle Kontamination formuliert werden.

Die mikrobielle Stabilität wird bislang durch den Zusatz an Konservierungsmitteln gelöst.

Bekannte Konservierungsmittel sind die als Parabene bezeichneten Verbindungen, insbesondere 4-Hydroxybenzoesäure und deren Ester, Methylparaben, Ethylparaben, Propylparaben, Isopropylparaben, Butylparaben, Isobutylparaben, Phenylparaben.

Des Weiteren ist Phenoxyethanol, Ethylenglycolmonophenylether, Phenylglycol, Phenoxetol®, zur Konservierung von kosmetischen Mitteln bekannt.

Zubereitungen ohne oder möglichst geringer Menge an Konservierungsmittel bereit zu stellen ist jedoch ein Wunsch der Konsumenten.

Weitere bekannte Hilfsmittel zur mikrobiellen Stabilisierung sind Benzethoniumchlorid, Lauroylethylarginat, Octopirox und Methylisothiazolinon.

Benzethoniumchlorid, Benzyl-dimethyl-(4-{2-[4-(1,1,3,3-tetramethylbutyl)-phenoxy]-ethoxy}-ethyl)-ammoniumchlorid, der Struktur 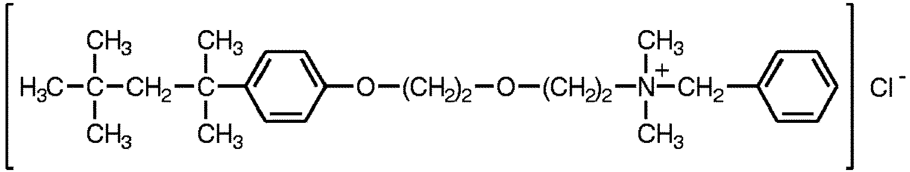 zeigt wie viele quartäre Ammonium-Verbindungen biozide Eigenschaften.

Als Desinfektionsmittel findet es Verwendung in Putzmitteln aber auch in Medikamenten (Spermizide, Lutschtabletten gegen Angina). Grapefruitkernextrakte können Benzethoniumchlorid in Konzentrationen von 7-11 % enthalten.

In Kosmetika ist der Einsatz von Benzethoniumchlorid bekannt, wie beispielweise in WO 2007015243 A1, WO 2004034964 A1, EP 1310234 A1, WO 2002008377 A1 beschrieben.

Laurylethylarginat, Lauroylethylarginat oder auch als Ethyl lauroyl arginate HCl bezeichnet, beispielsweise als Aminat G der Firma Vedeqsa, Inc. erhältlich, wird als Konservierungsmittel in kosmetischen Zubereitungen eingesetzt.

Aminat-G (INCI: Glycerin and ethyl lauroyl arginate HCl) zeigt ein großes antimikrobielles Spektrum und ist u.a. als Zusatz in Nahrungsmitteln FDA geprüft (siehe z.B. WO 2008133724 A1).

Piroctone Olamine, 1-Hydroxy-4-methyl-6-(2,4,4-trimethylpentyl)-2(1*H*)-pyridon, auch als Octopirox bezeichnet, ist ein Antimikrobikum, das als Konservierungsmittel verwendet und z. B. in Haarwaschmitteln als Antischuppenstoff eingesetzt wird. Octopirox ist gut hautverträglich und physiologisch indifferent.

### Octopirox hat die Struktur

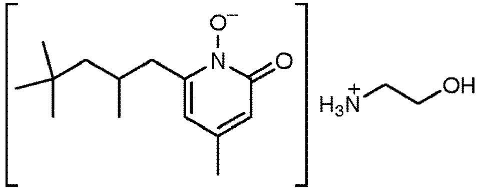

Methylisothiazolinone (2-Methyl-2*H*-isothiazol-3-on (MIT)) stellen eine Verbindungsklasse mit hoher mikrobizider Aktivität dar, die hauptsächlich zur Konservierung von technischen Produkten und nur wenigen kosmetischen Mitteln verwendet wird.

In kosmetischen Zubereitungen wird Methylisothiazolinon beispielsweise in US 6511673 oder EP 1488699 A1 in Kombination mit Parabenen bzw. Phenoxyethanol beschrieben. Weitere Verwendungen von Methylisothiazolinon werden in EP 1525797 A1, EP 1527684 A1, EP 1527685 A1, EP 1621076 A1 offenbart.

Ferner offenbart das Dokument EP 1153544 A2 Tränkungsmedien für Reinigungstücher, welche Benzethoniumchlorid enthalten. Das Dokument WO 02/069710 A1 offenbart konservierende Mischungen enthaltend quaternäre Ammoniumverbindungen. Unter anderem wird in den Mischungen Benzethoniumchlorid eingesetzt. Auch das Dokument WO 03/013454 A1 offenbart Konservierungsmittel und Mischungen von Konservierungsmittel für kosmetische Zubereitungen. Als Konservierungsmittel wird unter anderem Lauroylethylarginat in Konzentrationen von 0,2 Gew.-% eingesetzt. In gleicher maßen offenbart WO 2006125098 A2 von Lauroylethylarginat. US 2007/0082018 A1 offenbart ebenfalls Zubereitungen enthaltend Lauroylethylarginat. Hinsichtlich zunehmender Gesundheitsbeeinträchtigungen und Allergien sowie dem Drang der Konsumenten nach mehr ökologisch verträglichen Kosmetika besteht das Problem, dass diese Zubereitungen dennoch eine ausreichende Stabilität und vor allem Hautverträglichkeit aufweisen müssen.

Des Weiteren ist ein einfacher Austausch oder das Vermeiden von Parabenen und/oder Phenoxyethanol ohne Einbußen hinsichtlich der Stabilität und Anwendungseigenschaft nicht ohne weiteres möglich.

Weiteres Problem bei der Herstellung kosmetischer Zubereitungen zeigt sich bei der Kombination mit Wirkstoffen, insbesondere bei anionisch oder kationisch geladenen Wirkstoffen, wie zuvor dargestellt. Weisen die Wirkstoffe eine gegenteilige Ladung als die verwendeten Stabilisierungshilfsmittel auf ist mit einer Instabilität und einem Wirkungsverlust zu rechnen, da sich beide Ladungen neutralisieren und unter Umständen sogar Komplexe bilden, die dann agglomerieren und ausflocken können.

Aufgabe ist es daher kosmetische oder dermatologische Zubereitungen bereit zu stellen, die den Einsatz anionisch oder kationisch geladener Wirkstoffe ohne Einbußen hinsichtlich des Wirkungspektrums ermöglicht. Zusätzliche Aufgabe ist, dass diese Zubereitungen auch mikrobiell stabil, haut-, haar- und anwendungsfreundlich formuliert sind.

Die Erfindung umfasst kosmetische oder dermatologische Zubereitungen umfassend Benzethoniumchlorid in Kombination mit einem oder mehreren anionisch geladenen Wirkstoffen, dadurch gekennzeichnet, dass UV-Filter mit anionischer Ladung als anionisch geladene Wirkstoffe enthalten sind, und wobei parabenfrei bzw. phenoxyethanolfrei bedeutet, dass der Anteil an Parabenen und/oder Phenoxyethanol unter 1 Gew. %, bezogen auf die Gesamtmasse der Zubereitung, liegt.

Der Anteil an Benzethoniumchlorid ist bevorzugt im Bereich von 0,01 bis 5 Gew.%, insbesondere im Bereich von 0,1 bis 2 Gew.%, bezogen auf die Gesamtmasse der Zubereitung zu wählen.

In der Zubereitung können vorteilhaft Benzethoniumchlorid und Lauroylethylarginat enthalten sein.

Der Anteil an Benzethoniumchlorid und Lauroylethylarginat ist bevorzugt im Bereich von 0,01 bis 5 Gew.%, insbesondere im Bereich von 0,1 bis 2 Gew.%, bezogen auf die Gesamtmasse der Zubereitung zu wählen.

Kationische Wirkstoffe sind solche die eine dauerhafte kationische Ladung tragen.

Anionische Wirkstoffe sind solche die eine dauerhafte anionische Ladung tragen.

In der Kosmetik häufig verwendete anionische Wirkstoffe sind Polysaccharide bzw. deren Derivate, z.B. Hyaluronsäure, Xanthangummi, und besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination, zu wählen.

Aber auch anionische Tenside und Säuren wie z.B.: Laurylethersulfat, Anissäure, Sobinsäure, Zitronensäure, Benzoesäure und deren Derivate, Dehydracetsäure, C8-C22 Fettsäuren, Stearinsäure, Dinatriumphenyldibenzimidazol Tetrasulfat, Terephthalidene Dicamphersulfonsäure, Salicylsäure und deren Derivate, Propionsäure, Ameisensäure und deren Derivate, 10-Undecylensäure und deren Derivate, Milchsäure, Äpfelsäure sowie Polyhydroxysäuren im Allgemeinen sind als anionische Wirkstoffe zu wählen.

In der Kosmetik häufig verwendete kationische Wirkstoffe sind kationische Polymere, wie beispielsweise kationische Cellulosederivate (z.B. Polymer JR 400® von Amerchol), kationische Stärke, Copolymere von Diallylammoniumsalzen und Acrylamiden, quaternisierte Vinylpyrrolidon/Vinyl-imadazol-Polymere (z.B. Luviquat® von der BASF), Kondensationsprodukte von Polyglycolen und Aminen, quaternisierte Kollagenpolypeptide (z.B. Lamequat® L von Grünau-Henkel), quaternisierte Weizenpolypeptide, Polyethylenimin, kationische Silikonpolymere, Copolymere der Adipinsäure mit Dimethylaminohydroxypropyldietylentriamin, Copolymere der Acrylsäure mit Dimethyldiallylamoniumchlorid (z.B. Merquat®550 von Chemviron), Polyaminopolyamide, kationische Chitinderivate, kationischer Guar-Gum (z.B. Jaguar® CBS von Hoechst Celanese), quaternisierte Ammoniumsalz-Polymere (z.B. Mirapol® AD-1 von Miranol) sowie kationische Biopolymere wie z.B. Chitosan (mittleres Molekulargewicht von 50.000 bis 2.000.000 g/mol [bestimmt mittels Gelpermeationschromatographie] und einem Deacylierungsgrad von 10 bis 99 % [bestimmt mittels ¹H-NMR]).

Chitosan stellt ein partiell deacyliertes Chitin dar. Dieses Biopolymer hat u.a. filmbildende Eigenschaften und zeichnet sich durch ein seidiges Hautgefühl aus. Von Nachteil ist jedoch seine starke Klebrigkeit auf der Haut, die insbesondere - vorübergehend - während der Anwendung auftritt. Entsprechende Zubereitungen können dann im Einzelfalle nicht vermarktungsfähig sein, da sie vom Verbraucher nicht akzeptiert bzw. negativ beurteilt werden. Chitosan wird bekanntermaßen beispielsweise in der Haarpflege eingesetzt. Es eignet sich, besser als das ihm zugrundeliegende Chitin, als Verdicker oder Stabilisator und verbessert die Adhäsion und Wasserresistenz von polymeren Filmen. Stellvertretend für eine Vielzahl von Fundstellen des Standes der Technik: H.P.Fiedler, "Lexikon der Hilfsstoffe für Pharmazie, Kosmetik und angrenzende Gebiete", dritte Auflage 1989, Editio Cantor, Aulendorf, S. 293, Stichwort "Chitosan".

Chitosan ist gekennzeichnet durch folgende Strukturformel: 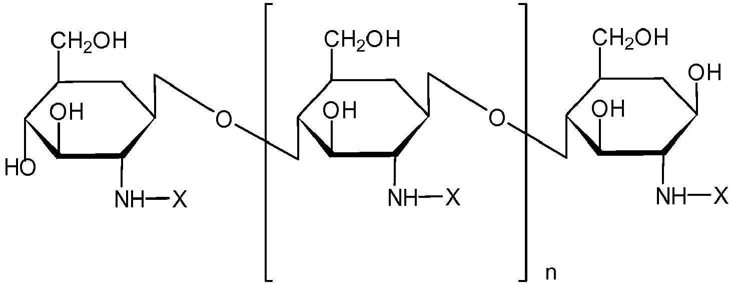 dabei nimmt n Werte bis zu ca. 10.000 an, X stellt entweder den Acetylrest oder Wasserstoff dar. Chitosan entsteht durch Deacetylierung und teilweise Depolymerisation (Hydrolyse) von Chitin, welches durch die Strukturformel 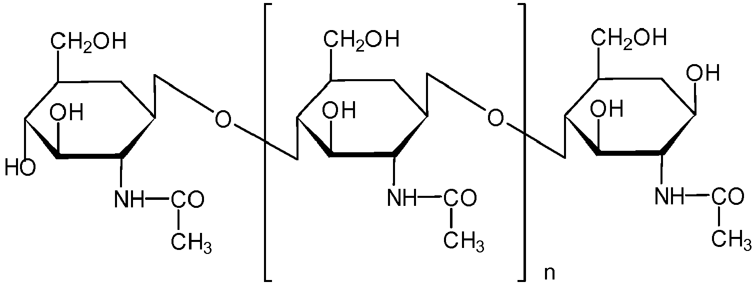 gekennzeichnet ist. Chitin ist wesentlicher Bestandteil des Ektoskeletts [το χιτων = grch.: der Panzerrock] der Gliederfüßer (z.B. Insekten, Krebse, Spinnen) und wird auch in Stützgeweben anderer Organismen (z.B. Weichtiere, Algen, Pilze) gefunden.

Im Bereich von etwa pH <6 ist Chitosan positiv geladen und dort auch in wässrigen Systemen löslich. Es ist nicht kompatibel mit anionischen Rohstoffen. Daher bietet sich zur Herstellung chitosanhaltiger Öl-in-Wasser-Emulsionen der Einsatz nichtionischer Emulgatoren an. Diese sind an sich bekannt, beispielsweise aus der EP-A 776 657.

Die Gesamtmenge an den kationischen Polymeren in den fertigen kosmetischen oder dermatologischen Zubereitungen wird vorteilhaft aus dem Bereich von 0,1 - 30 Gew.-%, bevorzugt 0,25 - 5,0 Gew.-% insbesondere 0,75 - 3,5 Gew.-% gewählt, bezogen auf das Gesamtgewicht der Zubereitungen.

Weitere kationische Stoffe sind Cetrimoniumchlorid, Behentrimoniumchlorid, Palmitamidopropyltrimonium Chloride, Distearyldimonium Chloride, Cocamidopropyl PG-Dimonium Chloride Phosphate, Quaternium-83, Hydroxyethyl Cetyldimonium Phosphate, Distearyldimonium Chloride, Behentrimonium Methosulfate, Cetrimonium Methosulfate, Quaternium-80, Stearalkonium Chloride, Cocotrimonium Methosulfate, sowie alle Subtanzen des Typus Polyquaternium-N mit N=1-111.

Als Wirkstoffe mit anionischer Ladung sind erfindungsgemäß UV-Filter wie Benzophenon-4 zu nennen.

Weitere Stoffe sind
- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Bisimidazylate (CAS-Nr.: 180898-37-7), welches beispielsweise unter der Handelsbezeichnung Neo Heliopan AP bei Haarmann & Reimer erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Haarmann & Reimer erhältlich ist;
- 1,4-di(2-oxo-10-Sulfo-3-bornylidenmethyl)-Benzol (auch: 3,3'-(1,4-Phenylendimethylene)-bis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird. Benzol-1,4-di(2-oxo-3-bornylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;
- Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Überraschend ist dabei weiterhin, dass die Zubereitungen mit den erfindungsgemäßen anionischen Stoffen und Benzethoniumchlorid, anstelle Parabenen und/oder Phenoyethanol dennoch eine ausreichende mikrobielle Stabilität aufweisen.

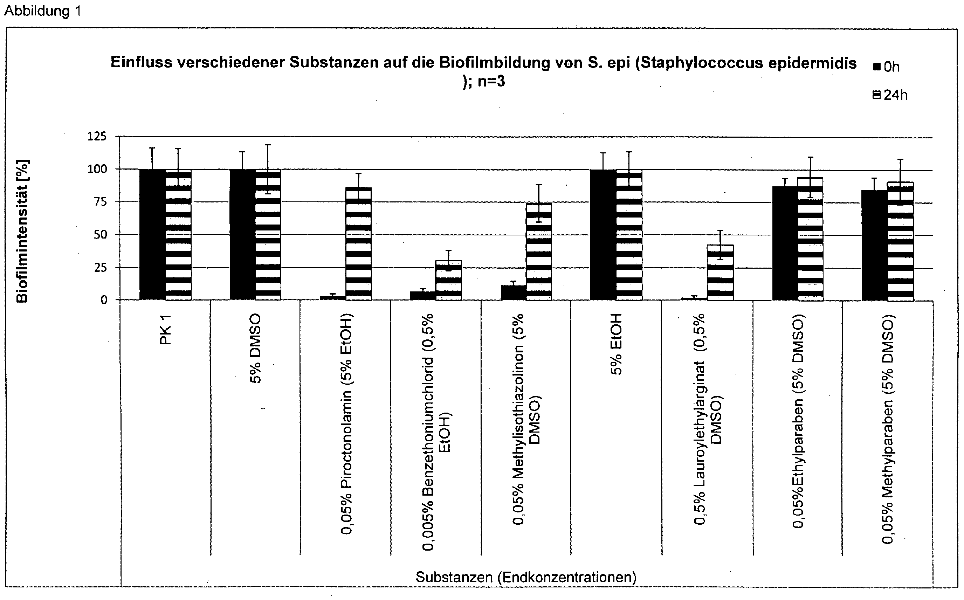

Abbildung 1 zeigt diese mikrobielle Stabilität eindrucksvoll.

Im Rahmen des Biofilmassays wird die Biofilm-Inhibition zum Zeitpunkt 0h wie auch die Biofilm-auflösende Wirkung zum Zeitpunkt 24h mittels einer Farbreaktion im Mikrotiterplattenmaßstab untersucht. Die Meßergebnisse der untersuchten Wirkstoffe werden auf die Positivkontrolle (PK1) bezogen. Weder Ethylparaben noch Methylparaben zeigten eine Wirksamkeit. Im Vergleich dazu zeigten Benzethoniumchlorid und Lauroylethylarginat eine sehr gute Biofilm-inhibierende Wirksamkeit. Darüber hinaus konnte für Benzethoniumchlorid und Lauroylethylarginat eine sehr gute Biofilm-auflösende Wirkung gezeigt werden.

Bei den herkömmlichen antibakteriellen Wirkstoffen unterscheidet man die bakteriostatischen und die bakterizide Wirkung. Bei letzterer werden die Bakterien durch den Wirkstoff abgetötet. Die bakteriostatische Wirkung dagegen verhindert die Vermehrung der Bakterien. Die in kosmetischen Formulierungen zur Verhinderung der Verkeimung eingesetzten Konservierungsmittel zählen in der Regel zu den bakteriziden Wirkstoffen. Durch den Einsatz klassischer antimikrobieller Wirker wird jedoch zum einen die Hautflora beeinträchtigt und zum anderen werden in den letzten Jahren zunehmend Resistenzentwicklungen beobachtet. Im Gegensatz zu herkömmlichen antimikrobiellen Wirkstoffen, greifen die Anti-Biofilm-Wirkstoffe nicht direkt in den bakteriellen Stoffwechsel ein, sondern verhindern lediglich die Bildung des die Bakterien schützenden Biofilms oder lösen diesen auf. Eine Resistenzentwicklung ist daher ausgeschlossen.

Der in Abbildung 1 dargestellte Test erfolgte mit Staphylococcus epidermidis, ein für Menschen mit normaler immunologischer Abwehr wenig gefährlicher Keim. Staphylococcus epidermidis (syn. Staphylococcus albus, Micrococcus epidermidis, Staphylococcus saprophyticus) ist ein grampositives, plasmakoagulase-negatives, saprophages Bakterium, das die menschliche Haut und Schleimhaut besiedelt. Des Weiteren ist er auf Lebensmitteln zu finden und siedelt auch auf polymeren Oberflächen (z.B. Verpackungen). Aber im Krankenhaus, bei abwehrgeschwächten Menschen, kann er, bei Unsauberkeit, Ursache für schwere Erkrankungen sein.

S. epidermidis besitzt ein großes Spektrum bei Antibiotikaresistenzen. Dies gilt vor allem gegen Penicillin und Methicillin. Der Anteil resistenter Stämme liegt mittlerweile bei 70%. Deshalb ist es das ideale Bakterium, um den vorteilhaften Effekt der erfindungsgemäßen kosmetischen Zubereitungen zu zeigen.

Die erfindungsgemäßen Zubereitungen sind Paraben- und/oder Phenoxyethanolfrei und weisen dennoch eine ausreichende mikrobielle Stabilität auf.

Die Kombination aus Benzethoniumchlorid mit einem oder mehreren anionisch geladenen Wirkstoffen zeigt wider Erwarten keinen Wirkungsverlust der Wirkstoffe.

Überraschenderweise lassen sich die gegensätzlich geladenen Stoffe, Benzethoniumchlorid sowie der oder die anionisch geladenen Wirkstoffen in einer Phase der Zubereitung einsetzen ohne dass es zu Instabilitäten oder Wirkverlusten kommt.

Erfindungsgemäß wird Benzethoniumchlorid in paraben- und/oder phenoxyethanolfreien kosmetischen oder dermatologischen Zubereitungen umfassend ein oder mehrere anionisch geladene Wirkstoffe dadurch gekennzeichnet, dass UV-Filter mit anionischer Ladung als anionisch geladene Wirkstoffe in der Zubereitung enthalten sind, und wobei parabenfrei bzw. phenoxyethanolfrei bedeutet, dass der Anteil an Parabenen und/oder Phenoxyethanol unter 1 Gew. %, bezogen auf die Gesamtmasse der Zubereitung, liegt,_zur Verbesserung der Stabilität der Zubereitung verwendet.

Verbesserte Stabilität bedeutet dabei, dass die Zubereitung mit den erfindungsgemäßen Stoffen gleiche oder bessere mikrobiologische Stabilitäten zeigen wie gleiche Zubereitungen ohne Benzethoniumchlorid. Ebenso ist unter verbesserter Stabilität die Beibehaltung oder Verbesserung der Wirkungsweise der anionischen Wirkstoffe zu verstehen im Vergleich zu den gleichen Zubereitungen ohne Benzethoniumchlorid.

Mit der verbesserten Stabilität geht auch eine verbesserte Lagerfähigkeit der Zubereitungen einher.

Diese vorteilhaften Eigenschaften wurden zum Beispiel mit der folgenden Rezeptur generiert:

| Zubereitungen A bzw. B | [Gew.%] |
|---|---|
| Wasser | 66.4100 |
| Octyldodecanol | 0.5000 |
| Cetearyl Alkohol | 2.0000 |
| EDTA aq | 1.0000 |
| Phenoxyethanol | 0.5000 |
| Cyclomethicon | 5.0000 |
| Myristylmyristat | 1.0000 |
| Butylmethoxydibenzoylmethan | 1.0000 |
| Phenylbenzimidazolesulfonsäure | 0.5000 |
| Glycerin | 7.0000 |
| Natronlauge aq | 0.3700 |
| Ethylhexylsalicylat | 4.5000 |
| Acrylates/C 10-30 Alkyl Acrylate Crosspolymer | 0.2000 |
| Octocrylene | 2.0000 |
| Tapioca Stärke aq | 4.0000 |
| Ammonium Acryloyldimethyltaurat/VP Copolymer | 0.4700 |
| Methylpropanediol | 3.0000 |
| Natriumstearoylglutamat | 0.2000 |
| Benzethoniumchlorid | 0.0500 |
| Parfum | 0.3000 |

A= Einsatz von Benzethoniumchlorid und Phenylbenzimidazolesulfonsäure gemeinsam in die Wasserphase
B= Getrennter Einsatz von Benzethoniumchlorid und Phenylbenzimidazolesulfonsäure in zwei verschiedenen Wasserphasen.

### Messmethode:

Der SPF (Sun Protection Factor / Lichtschutzfaktor) wird nach den Vorschriften der New International SPF Method (Februar 2003) in vivo auf menschlicher Haut bestimmt.

Definition: SPF = Quotient aus minimaler erythemaler Dosis (MED) auf geschützter und ungeschützter Haut.

Die MED ist die niedrigste Dosis von UV-Strahlung, die nach 16-24 h eine schwache, aber deutlich erkennbare Hautrötung (Sonnenbrand, Erythem) hervorruft. Strahlungsquellen sind Sonnen-Simulatoren, meistens mit Xenon-Glühlampen.

Der SPF-Wert, ein Wert zum Nachweis der UV-Lichtschutzwirkung, ist bei beiden Zubereitungen ähnlich, A hatte in der ersten Messung einen SPF von 14,6 und B von 14,6.

D.h. die Kombination von Benzethoniumchlorid sowie der oder die anionisch geladenen Wirkstoffen in einer Phase der Zubereitung führen zu keinem Wirkverlust, hier der SPF.

Darüber hinaus wurde dieser Effekt auch in der mikrobiologischen Belastung mit unterschiedlichen Keimen bestätigt:
A= Einsatz von Benzethoniumchlorid und Phenylbenzimidazolesulfonsäure gemeinsam in die Wasserphase

| Keim | Ausgang | nach 7 Tagen | nach 14 Tagen | nach 28 Tagen |
|---|---|---|---|---|
| Pseudomonas putida/cepacia | 3,00E+05 | 1,00E+02 | 1,00E+02 | 1,00E+02 |
| Escherichia Coli/Enterob. gergoviae | 3,00E+05 | 1,00E+02 | 1,00E+02 | 1,00E+02 |
| Aspergillus Brasiliensis | 2,60E+05 | 5,00E+03 | 1,00E+02 | 1,00E+02 |
| Candida albicans/parapsilosis/guilliermondii | 2,30E+05 | 1,10E+03 | 1,00E+02 | 1,00E+02 |

B= Getrennter Einsatz von Benzethoniumchlorid und Phenylbenzimidazolesulfonsäure zwei verschiedenen Wasserphasen.

| Keim | Ausgang | nach 7 Tagen | nach 14 Tagen | nach 28 Tagen |
|---|---|---|---|---|
| Pseudomonas putida/cepacia | 3,00E+05 | 1,00E+02 | 1,00E+02 | 1,00E+02 |
| Escherichia Coli/Enterob. gergoviae | 3,00E+05 | 1,00E+02 | 1,00E+02 | 1,00E+02 |
| Aspergillus Brasiliensis | 2,60E+05 | 5,00E+03 | 1,00E+02 | 1,00E+02 |
| Candida albicans/parapsilosis/guilliermondii | 2,30E+05 | 1,10E+03 | 1,00E+02 | 1,00E+02 |

Die Absterbekinetik ist identisch, das zeigt eindrucksvoll, dass das kationische Konservierungsmittel Benzethoniumchlorid nicht durch den anionischen UV-Filter deaktiviert worden ist.

Die erfindungsgemäßen Zubereitungen sind Paraben- und/oder Phenoxyethanolfrei und weisen dennoch eine ausreichende mikrobielle Stabilität auf.

Parabenfrei bzw. Phenoxyethanolfrei bedeutet, dass der Anteil an Parabenen und/oder Phenoxyethanol unter 1 Gew.% liegt, bezogen auf die Gesamtmasse der Zubereitung. Vorteilhaft ist der Anteil an Parabenen und Phenoxyethanol 0 Gew.%.

Die kosmetischen Zubereitungen gemäß der Erfindung können ferner kosmetische Hilfsstoffe und weitere Wirkstoffe enthalten, wie sie üblicherweise in solchen Zubereitungen verwendet werden, z. B. Substanzen zum Verhindern des Schäumens, Farbstoffe und Farbpigmente, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate sofern der Zusatz die geforderten Eigenschaften hinsichtlich der mikrobiologischen Stabilität sowie Haut- und Haarverträglichkeit nicht beeinträchtigt.

Die erfindungsgemäßen Zubereitungen können vorteilhaft in Form von wässrigen Gelen, wässrig-alkoholischen Lösungen, O/W- oder W/O-Emulsionen von weicher, halb-weicher oder fester Konsistenz, Mikroemulsionen, W/O/W- oder O/W/O-Emulsionen. Ideale Applikationsformen sind in diesem Zusammenhang Gesichtscremes, Seren, Make-up, Foundation, Body Lotions, Body Milks, Handcreme, Deo-Roller, Deo-Stick, Deo-Aerosol, Deo-Zerstäuber.

Auch der Einsatz in rinse-off-Produkten wie Shampoos, Duschgelen oder Handwaschgelen sind bevorzugt.

Die nachfolgenden Beispiele zeigen kosmetische Zubereitungen mit uneingeschränkter Wirkleistung.

Die Zahlenangaben sind Gewichtsanteile bezogen auf die Gesamtmasse der Zubereitung.

### Hydrodispersionsgel

Die nachfolgenden Beispiele sind keine erfindungsgemäßen Hydrodispersionsgele.

| | 1 | 2 | 3 | 4 | 5 |
|---|---|---|---|---|---|
| Silikonöl, cyclisch | 8 | 10 | 5 | 3 | --- |
| Silikonöl, linear | --- | --- | --- | --- | 3 |
| Dimethiconol | 1 | 2 | 3 | --- | 3 |
| Ethanol | 1,0 | 5,0 | 7,5 | 1,5 | 3,0 |
| Natriumpolyacrylat | 0,2 | 0,3 | 0,3 | 0,4 | 0,10 |
| Methylpropandiol | 2 | 3 | 4 | 5 | --- |
| Glycerin | 9 | 15 | 5 | 7,5 | 25 |
| Carbomer | 0,2 | 0,3 | 0,2 | 0,4 | 0,15 |
| Acrylates/C10-30Alkyl Acrylate Crosspolymer | 0,2 | 0,15 | 0,3 | 0,4 | 0,10 |
| Carrageenan (Chondrus Crispus) | --- | --- | --- | --- | 2 |
| Hamamelis Extrakt | 0,1 | 0,2 | 0,3 | 0,4 | --- |
| Parfum | q.s. | q.s. | q.s. | q.s. | q.s. |
| Lauroylethylarginat | 0,5 | --- | 0,5 | --- | 0,1 |
| Benzethoniumchlorid | 0,5 | 1,5 | --- | 0,1 | --- |
| Wasser | ad 100 | ad 100 | ad 100 | ad 100 | ad 100 |

### O/W-Emulsion

Die nachfolgenden Beispiele sind keine erfindungsgemäßen O/W-Emulsionen.

| | 6 | 7 | 8 | 9 | 10 |
|---|---|---|---|---|---|
| Glycerylstearatcitrat | 2,4 | 2,0 | 2,0 | 2,0 | 2,4 |
| Medizinisches Weissöl | --- | 6,0 | 6,0 | 6,0 | --- |
| Myristylmyristat | 3,0 | --- | --- | --- | 3,0 |
| Isopropylpalmitat | --- | 3,0 | 3,0 | 3,0 | --- |
| Caprylic/Capric Triglycerid | 4,0 | --- | --- | --- | 4,0 |
| lineares Silikonöl | 2,0 | 1,0 | 1,0 | 1,0 | 2,0 |
| Sorbitanstearat | 0,8 | --- | --- | --- | 0,8 |
| Octyldodecanol | 1,0 | --- | --- | --- | 1,0 |
| Cetearylalkohol | 0,8 | 2,0 | 2,0 | 2,0 | 0,8 |
| Glycerin | 6,5 | --- | --- | --- | 6,5 |
| Polyacrylsäure, Natrium-Salz | 0,19 | 0,19 | 0,19 | 0,19 | 0,19 |
| Xanthan Gum | 0,2 | --- | --- | --- | 0,2 |
| Ethanol | 3,0 | --- | --- | --- | 3,0 |
| Parfum | 0,3 | 0,3 | 0,3 | 0,3 | 0,6 |
| Lauroylethylarginat | 1,0 | --- | 0,5 | 0,5 | 0,25 |
| Benzethoniumchlorid | 1,0 | 2,0 | --- | 0,5 | --- |
| Wasser | ad 100 | ad 100 | ad 100 | ad 100 | ad 100 |

### W/O-Emulsionen

Die nachfolgenden Beispiele sind keine erfindungsgemäßen W/O Emulsionen.

| | 1 | 2 | 3 | 4 | 5 |
|---|---|---|---|---|---|
| Triglycerindiisostearat | 1,0 | 0,5 | 0,25 | 2,0 | 3,0 |
| Diglycerindipolyhydroxystearat | 1,0 | 1,5 | 1,75 | 3,0 | 2,0 |
| Paraffinöl | 12,5 | 10,0 | 8,0 | 5,0 | 11,5 |
| Vaseline | 8,0 | 6,0 | 5,0 | 12,0 | 2,5 |
| hydrierte Kokosglyceride | 2,0 | 1,0 | 2,5 | 5,0 | 0,25 |
| Decyloleat | 0,5 | 0,75 | 1,0 | 2,0 | 0,25 |
| Octyldodecanol | 0,5 | 1,0 | 0,75 | 3,0 | 0,25 |
| Aluminiumstearat | 0,4 | 0,3 | 0,6 | 1,0 | 0,05 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Chitosan | 0,5 | 1,0 | 0,25 | --- | --- |
| Polyacrylsäure, Na-Salz | --- | --- | --- | 0,15 | 0,25 |
| Magnesiumchlorid | 0,5 | 0,6 | 0,5 | 0,7 | 1,0 |
| Glycerin | 3,0 | 5,0 | 10,0 | 15,0 | 1,5 |
| Milchsäure | 0,5 | 0,9 | 0,2 | --- | --- |
| Parfum | q,s, | q,s, | q,s, | q,s, | q,s, |
| Ethanol | 2,0 | --- | 5,0 | --- | --- |
| Capryl-/Caprinsäuretriglycerid | 2,0 | 2,5 | 3,0 | 5,0 | 0,5 |
| Lauroylethylarginat | 1,0 | --- | 0,5 | 0,5 | 0,25 |
| Benzethoniumchlorid | 1,0 | 2,0 | --- | 0,5 | --- |
| Kaliumsorbat | 0,04 | 0,15 | 0,05 | 0,03 | 0,4 |
| Wasser | ad 100 | ad 100 | ad 100 | ad 100 | ad 100 |

O/W Emulsion: Die Nachfolgenden W/O Emulsionen 1-4 sind nicht erfindungsgemäß

| | 1 | 2 | 3 | 4 | 5 |
|---|---|---|---|---|---|
| Panthenol | 1,5 | 0 | 0 | 0,7 | 0 |
| Butylenglykol | --- | --- | --- | 5 | 10 |
| Propylenglykol | --- | --- | 10 | 5 | --- |
| Benzethoniumchlorid | 1,0 | 0,05 | 0,25 | 0,5 | 0,9 |
| Lauroylethylarginat | 1,0 | 2,0 | 1,5- | 1,0 | 0,5 |
| medizinisches Weissöl | 0 | 6 | 6 | 0 | 0 |
| Isopropylpalmitat | 1 | 0 | 0 | 0 | 0 |
| Caprylic/Capric Triglycerid | 0 | 3 | 3 | 0 | 1,00 |
| Cetearylalkohol | 0 | 0 | 0 | 2,5 | 4,00 |
| Cetylalkohol | 0 | 3 | 3 | 0 | 0 |
| lineares Silikonöl | 0 | 3 | 3 | 0 | 0,50 |
| cyclisches Silikonöl | 0 | 3 | 3 | 0 | 0 |
| C12-15 Alkylbenzoat | 0 | 0 | 0 | 0 | 2,00 |
| Sheabutter | 0 | 0,5 | 0,5 | 0 | 0 |
| Dicaprylylether | 0 | 0 | 0 | 10 | 0 |
| Dicaprylylcarbonat | 2 | 0 | 0 | 0 | 0 |
| Glyceryl stearat | 0 | 1,2 | 1,2 | 2,4 | 2,4 |
| Tapiocastärke | 1 | 0 | 0 | 0 | 0 |
| Glycerin | 5 | 8 | 10 | 8 | 8 |
| Butylenglykol | 0 | 0 | 0 | 1 | 0 |
| Zitronensäure | 0,0050 | 0 | 0 | 0 | 0 |
| Natronlauge 45% | 0,35 | 0,01 | 0,01 | 0,25 | 1 |
| Polyglyceryl-10 Myristat | 0 | 1,8 | 0 | 0 | 0 |
| Polyglyceryl-10 Stearat | 0,7 | 0 | 1,8 | 1 | 1 |
| Polyacrylsäure, Na-Salz (Carbopol 981) | 0,5 | 0,02 | 0,02 | 0 | 0 |
| Acrylic Acid/VP Crosspolymer | 0 | 0 | 0 | 0,5 | 0,75 |
| Trinatrium EDTA | 0 | 1 | 1 | | 1 |
| Ethylhexylmethoxycinnamat | 0 | 0 | 0 | 2 | 0 |
| Butylmethoxydibenzoylmethan | 0 | 0 | 0 | 0 | 2 |
| Phenylbenzimidazolesulfonsäure, Natrium-Salz | 0 | 0 | 0 | 0 | 2 |
| Ethylhexylsalicylat | 0 | 0 | 0 | 0 | 2 |
| Titandioxid, silikongecoated | 0 | 0 | 0 | 0,3600 | 0 |
| Octocrylen | 0 | 0 | 0 | 0 | 2 |
| Parfum | 0,10 | 0,35 | 0,35 | 0 | 0,20 |
| Wasser | ad 100 | ad 100 | ad 100 | ad 100 | ad 100 |

## Patentansprüche

1. Kosmetische oder dermatologische, paraben- und/oder phenoxyethanolfreie Zubereitung umfassend Benzethoniumchlorid in Kombination mit einem oder mehreren anionisch geladenen Wirkstoffen, **dadurch gekennzeichnet, dass** UV-Filter mit anionischer Ladung als anionisch geladene Wirkstoffe enthalten sind, und wobei parabenfrei bzw. phenoxyethanolfrei bedeutet, dass der Anteil an Parabenen und/oder Phenoxyethanol unter 1 Gew. %, bezogen auf die Gesamtmasse der Zubereitung, liegt.

2. Zubereitung nach Anspruch 1, **dadurch gekennzeichnet, dass** Lauroylethylarginat enthalten ist.

3. Zubereitung nach einem der vorhergehenden Ansprüche, **dadurch gekennzeichnet, dass** der Anteil an Benzethoniumchlorid im Bereich von 0,01 bis 5 Gew.%, insbesondere im Bereich von 0,1 bis 2 Gew.%, bezogen auf die Gesamtmasse der Zubereitung, gewählt wird.

4. Zubereitung nach Anspruch 2, **dadurch gekennzeichnet, dass** der Anteil an Benzethoniumchlorid und Lauroylethylarginat im Bereich von 0,01 bis 5 Gew.%, insbesondere im Bereich von 0,1 bis 2 Gew.%, bezogen auf die Gesamtmasse der Zubereitung, gewählt wird.

5. Verwendung von Benzethoniumchlorid in paraben- und/oder phenoxyethanolfreien kosmetischen oder dermatologischen Zubereitungen umfassend ein oder mehrere anionisch geladene Wirkstoffe **dadurch gekennzeichnet, dass** UV-Filter mit anionischer Ladung als anionisch geladene Wirkstoffe in der Zubereitung enthalten sind, und wobei parabenfrei bzw. phenoxyethanolfrei bedeutet, dass der Anteil an Parabenen und/oder Phenoxyethanol unter 1 Gew. %, bezogen auf die Gesamtmasse der Zubereitung, liegt, zur Verbesserung der Stabilität der Zubereitung.

## Claims

1. Cosmetic or dermatological, paraben-free and/or phenoxyethanol-free preparation comprising benzethonium chloride in combination with one or more anionically charged active ingredients, **characterized in that** UV filters with anionic charge are present as anionically charged active ingredients, and wherein paraben-free and/or phenoxyethanol-free signifies that the fraction of parabens and/or phenoxyethanol is below 1% by weight, based on the total mass of the preparation.

2. Preparation according to Claim 1, **characterized in that** ethyl lauroyl arginate is present.

3. Preparation according to either of the preceding claims, **characterized in that** the fraction of benzethonium chloride is selected in the range of 0.01 to 5% by weight, especially in the range of 0.1 to 2% by weight, based on the total mass of the preparation.

4. Preparation according to Claim 2, **characterized in that** the fraction of benzethonium chloride and ethyl lauroyl arginate is selected in the range of 0.01 to 5% by weight, especially in the range of 0.1 to 2% by weight, based on the total mass of the preparation.

5. Use of benzethonium chloride in paraben-free and/or phenoxyethanol-free cosmetic or dermatological preparations comprising one or more anionically charged active ingredients, **characterized in that** UV filters with anionic charge are present in the preparation as anionically charged active ingredients, and wherein paraben-free and/or phenoxyethanol-free signifies that the fraction of parabens and/or phenoxyethanol is below 1% by weight, based on the total mass of the preparation, for improving the stability of the preparation.

## Revendications

1. Préparation cosmétique ou dermatologique sans parabènes et/ou sans phénoxyéthanol, comprenant du chlorure de benzéthonium en combinaison avec un ou plusieurs agents actifs chargés anioniquement, **caractérisée en ce que** des filtres UV à charge anionique sont contenus en tant qu'agents actifs chargés anioniquement, et sans parabènes et sans phénoxyéthanol signifie que la proportion de parabènes et/ou de phénoxyéthanol est inférieure à 1 % en poids, par rapport à la masse totale de la préparation.

2. Préparation selon la revendication 1, **caractérisée en ce que** de l'arginate de lauroyléthyle est contenu.

3. Préparation selon l'une quelconque des revendications précédentes, **caractérisée en ce que** la proportion de chlorure de benzéthonium est choisie dans la plage allant de 0,01 à 5 % en poids, notamment dans la plage allant de 0,1 à 2 % en poids, par rapport à la masse totale de la préparation.

4. Préparation selon la revendication 2, **caractérisée en ce que** la proportion de chlorure de benzéthonium et d'arginate de lauroyléthyle est choisie dans la plage allant de 0,01 à 5 % en poids, notamment dans la plage allant de 0,1 à 2 % en poids, par rapport à la masse totale de la préparation.

5. Utilisation de chlorure de benzéthonium dans des préparations cosmétiques ou dermatologiques sans parabènes et/ou sans phénoxyéthanol comprenant un ou plusieurs agents actifs chargés anioniquement, **caractérisée en ce que** des filtres UV à charge anionique sont contenus dans la préparation en tant qu'agents actifs chargés anioniquement, et sans parabènes et sans phénoxyéthanol signifie que la proportion de parabènes et/ou de phénoxyéthanol est inférieure à 1 % en poids, par rapport à la masse totale de la préparation, pour l'amélioration de la stabilité de la préparation.
